# EUROPÄISCHE PATENTANMELDUNG

(11) **EP 0 976 721 A2**
(43) Veröffentlichungstag der Anmeldung: **02.02.2000**
(21) Anmeldenummer: 99114037.7
(22) Anmeldetag: 20.07.1999
(51) Int. Cl.: C07C 227/32

(54) **Verfahren zur Herstellung von Aminosäuren und Aminosäurederivaten**

(30) Priorität: 28.07.1998 DE 19833853
(71) Anmelder: Degussa-Hüls Aktiengesellschaft, 60311 Frankfurt am Main (DE)
(72) Erfinder: Vogt, Annegret, Dr., 63450 Hanau (DE); Altenbach, Hans-Josef Prof., 42119 Wuppertal (DE); Kirschbaum, Michael, 42119 Wuppertal (DE); Hahn, Michael Gottfried, 50937 Köln (DE); Matthäus, Mike Siegfried Paul, 42327 Wuppertal (DE); Hermann, Andreas Rainer, 42119 Wuppertal (DE)

(57) **Zusammenfassung**

Die vorliegende Erfindung richtet sich auf ein chemisches Verfahren zur Herstellung von Verbindungen der allgemeinen Formel (I) 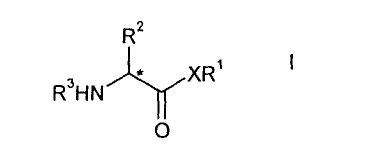 ausgehend von Verbindungen der allgemeinen Formel (II) und (III) 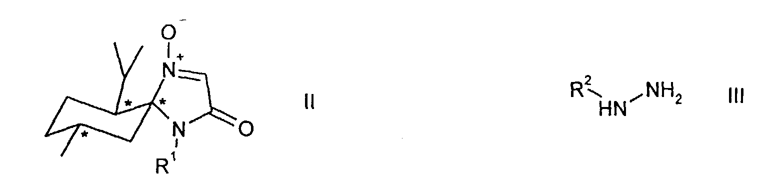 unter radikalischen Bedingungen. Die Produkte I werden als Intermediate in der Synthese bioaktiver Wirkstoffe eingesetzt.

## Beschreibung

Die vorliegende Erfindung richtet sich auf ein Verfahren zur Herstellung enantiomerenangereicherter Aminosäuren und Aminosäurederivaten der allgemeinen Formel (I) oder Säureadditionssalzen hiervon 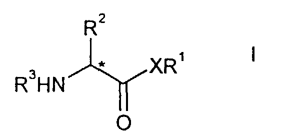 worin
* = Asymmetriezentrum
   X = O oder NH
R¹ = H, (C₁-C₆) Alkyl, Benzyl oder
   (C₃-C₆) Alkoxycarbonylmethyl und
R² = H, (C₁-C₆) Alkyl, das mit Heteroatomen wie N, P, O, S oder Si unterbrochen oder substituiert sein kann, wobei die Heteroatome selbst ein- oder mehrfach mit linear oder verzweigten (C₁-C₃) Alkyl substituiert sein können, (C₂-C₆) Alkenyl, (C₁-C₆) Haloalkyl, Halogen, Aryl, wie Naphthyl oder Phenyl, welche ein- oder mehrfach mit (C₁-C₃) Alkyl, Hydroxy, Halogen oder (C₁-C₃) Alkoxy substituiert sein können, Aralkyl wie 2-Naphthylmethyl oder Benzyl oder 1,1- oder 1,2-Phenethyl, welche seinerseits ein- oder mehrfach mit (C₁-C₃) Alkyl, Hydroxy, Halogen oder (C₁-C₃) Alkoxy substituiert sein können, Heteroaralkyl wie N-geschütztes 3-Indolylmethyl
R³ = H, OH bedeutet,
aus enantiomerenangereicherten Nitronen der allgemeinen Formel (II) 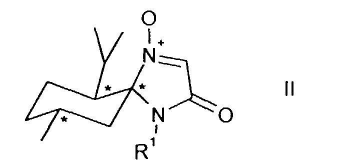 worin *, R¹ die oben angegebene Bedeutung besitzen.

Enantiomerenangereicherte Aminosäuren und Aminosäurederivate sind wichtige Substanzen in der organischen Synthese von Peptiden und Peptidmimetika, welche in Arzneimitteln und bioaktiven Wirkstoffen Anwendung finden. So benötigt man das nach dem erfindungsgemäßen Verfahren herstellbare optisch aktive tert.-Leucinmethylamid in der Synthese zur Herstellung eines Matrixmetalloproteinaseinhibitors, welcher zur Zeit in der klinischen Phase zwecks Bekämpfung von Tumoren untersucht wird (J. Med. Chem. 1998, 41, 1209-1217).

Aus der WO 97/10203 ist ein Verfahren zur Addition von Nucleophilen an Nitrone der allgemeinen Formel (II) bekannt. Das genannte Verfahren basiert jedoch auf dem Einsatz von starken Basen wie Alkylmetallverbindungen. Man ist deshalb auf den Einsatz von absolutierten Lösungsmitteln und das Arbeiten unter Schutzgas sowie jeglichen Ausschluß von Wasserspuren bei der Reaktion beschränkt. Ein solches Verfahren ist im industriellen Maßstab nur schwer durchzuführen.

Ebenfalls in der WO 97/10203 wird ein Verfahren zur Addition radikalischer Verbindungen an Derivate der allgemeinen Formel (II) bereits erwähnt. Allerdings wurde dort die Umsetzung der Derivate (II) mit einer Carbonsäure in Gegenwart eines Radikalstarters in verschiedenen Lösungsmitteln unter Ausschluß von Sauerstoff beschrieben. Unter den gewählten Bedingungen erhält man jedoch nach der Umsetzung α-substituierte Nitrone ohne die eigentlich erwünschte Asymmetrie am α-C-Atom des Aminosäurebausteins zurück.

Laut Iwamura et al. (Bull. Chem. Soc. Jpn. 1970, 43, 856-860) erhält man bei der Umsetzung von Nitronen mit Radikalen 1,3-Additionsprodukte oder Nitroxide bzw. substituierte Nitrone. In bestimmten Fällen disproportioniert das substituierte Nitroxid in Nitron und Hydroxylamin (Iwamura et al. Bull Chem. Soc. Jpn. 1967, 40, 703). Die Produkte wurden nicht isoliert, doch erlaubt dieser Mechanismus lediglich eine Ausbeute von max. 50 % an gewünschter Verbindung. 50 % der Produktverbindungen fallen als Abfall an, was technisch gesehen als wenig sinnvolle Produktvariante in Frage kommt.

Alle bisher beschriebenen Verfahrensvarianten zur Addition von Verbindungen an Nitrone der Formel (II) (s. WO 97/10203) haben die Nachteile, daß sie insbesondere bei sterisch anspruchsvollen Aminosäuren nur moderate Ausbeuten liefern und zum Teil einer aufwendigen Reaktionsführung bedürfen (metallorganische Reagenzien, Wasserausschluß, Reinigung etc.).

Aufgabe der vorliegenden Erfindung ist die Angabe eines Verfahrens zur Herstellung von enantiomerenangereicherten Aminosäuren und Aminosäurederivaten unter radikalischen Bedingungen ausgehend von den Nitronen (II) unter Erhalt eines am α-C-Atom des Aminosäurebausteins befindlichen stereogenen Zentrums.

Unter radikalischen Bedingungen wird im Rahmen der Erfindung eine Reaktion verstanden, bei der zumindest einer der Reaktanden Radikalcharakter hat.

Diese und nicht näher genannte weitere Aufgaben, welche sich jedoch aus dem Stand der Technik in naheliegender Weise ergeben, werden durch die kennzeichnenden Merkmale des Anspruchs 1 gelöst.

Dadurch, daß man zur Herstellung enantiomerenangereicherter Aminosäuren und Aminosäurederivaten der allgemeinen Formel (I) oder Säureadditionssalzen hiervon 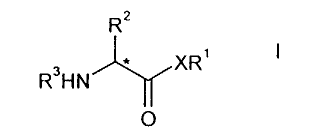 worin
* = Asymmetriezentrum
   X = O oder NH
R¹ = H, (C₁-C₆) Alkyl, Benzyl oder
   (C₃-C₆) Alkoxycarbonylmethyl und
R² = H, (C₁-C₆) Alkyl, das mit Heteroatomen wie N, P, O, S oder Si unterbrochen oder substituiert sein kann, wobei die Heteroatome selbst ein- oder mehrfach mit (C₁-C₃) Alkyl substituiert sein können, (C₂-C₆) Alkenyl, (C₁-C₆) Haloalkyl, Halogen, Aryl, wie Naphthyl oder Phenyl, welche ein- oder mehrfach mit (C₁-C₃) Alkyl, Hydroxy, Halogen oder (C₁-C₃) Alkoxy substituiert sein können, Aralkyl wie 2-Naphthylmethyl oder Benzyl oder 1,1- oder 1,2-Phenethyl, welche seinerseits ein- oder mehrfach mit (C₁-C₃) Alkyl, Hydroxy, Halogen oder (C₁-C₃) Alkoxy substituiert sein können, Heteroaralkyl wie N-geschütztes 3-Indolylmethyl
R³ = H, OH bedeutet,
diastereomerenangereicherte Nitrone der allgemeinen Formel (II) 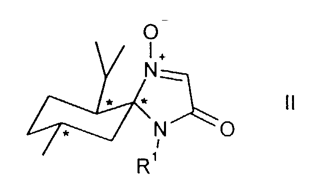 worin *, R¹ die oben angegebene Bedeutung besitzen, dergestalt umsetzt, daß man das Nitron der allgemeinen Formel (II) mit einem Hydrazinderivat der allgemeinen Formel (III) 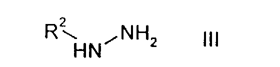 wobei R² die oben angegebene Bedeutung besitzt, in einem Lösungsmittel in Gegenwart eines Radikalstarters oder unter elektrochemischen Bedingungen zu Verbindungen der allgemeinen Formel (IV) 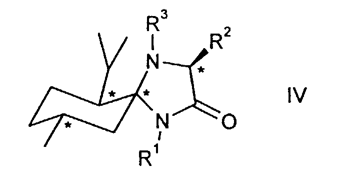 worin R¹ und R² die oben angegebene Bedeutung annehmen und R³ = OH ist, umsetzt, anschließend hydrolysiert oder erst zu Verbindungen der allgemeinen Formel (IV), worin R¹ und R² die oben angegebene Bedeutung annehmen und R³ = H ist, reduziert und dann hydrolysiert, gelangt man in ganz außerordentlich einfacher und eleganter Weise zu den gewünschten Verbindungen.

Die elektrochemische Radikalgenerierung aus Verbindungen des Typs III kann nach dem Fachmann bekannte Methoden erfolgen (z. B.: F. Beck, Elektroorg. Chemie 1974, VCH-Verlag; T. Shono, Electroorganic Synthesis, 1991, Academic Press; T. Shono, Electroorganic Chemistry as a New Tool in Organic Synthesis 1984, Springer Verlag). Als Elektrodenmaterial kommen Kohle, aber auch Metalle wie Silber und Platin in Frage. Als Lösungsmittel können Carbonsäureester und Alkohole, bevorzugt Essigester und Methanol, sowie Mischungen dieser eingesetzt werden.

Die radikalische Addition kann bei Temperaturen zwischen -78 °C und 150 °C, bevorzugt -20 °C bis 100 °C und besonders bevorzugt bei -10 °C bis 50 °C, erfolgen. Die Reaktionskontrolle erfolgt mittels Dünnschicht- oder Gaschromatographie. Das Ende der Reaktion ist auch an der nachlassenden Gasentwicklung zu beobachten.

Man kann bei dieser Reaktion in einem organischen Lösungsmittel, wie einem halogenierten Kohlenwasserstoff, wie Methylenchlorid, Trichlormethan oder Dichlorethan, in Estern, wie Essigester, Ethern, wie Diethylether, MtBE, THF, Dioxan, oder Alkoholen, wie Methanol, Ethanol, Propanol, Isopropanol, n-, sec-, Iso-, tert.-Butanol, sowie einem inerten aromatischen Kohlenwasserstoff, wie Benzol, Toluol, Xylol, Chlorbenzol, Nitrobenzol ggf. in Gegenwart von Wasser arbeiten.

Besonders bevorzugt ist der Einsatz eines Zweiphasensystems aus Toluol und Wasser.

Als Radikalstarter können im Prinzip alle dem Fachmann bekannten Derivate, die für diesen Zweck in Frage kommen, genommen werden. Gängige Radikalstarter sind beispielsweise Bleidioxid, Natrium- oder Kaliumperoxodisulfat, Eisen-(III)-Salze, Natriumnitrit, Wasserstoffperoxid, Natriumperiodat, Natriumhypochlorit, Natriumperborat, Natriumpercarbonat oder meta-Chlorperbenzoesäure (siehe auch Houben-Weyl "Methoden der Organischen Chemie", Bd. E19a, Teil 1, S. 140ff, sowie Teil 2, z.B. S. 1201ff; Bd. E16a, S. 805ff; Bd. X/2, S. 68ff, E. J. Corey; A. W. Gross, J. Org. Chem. 1985, 50, 5391).

Besonders bevorzugt ist der Einsatz von Oxidationsmitteln wie Natrium- oder Kaliumperoxodisulfat, Natriumpercarbonat bzw. Natriumperborat.

Die Reduktion der Hydroxylamine (IV) mit R³ = OH zu den entsprechenden Aminen mit R³ = H kann in an sich bekannter Weise erfolgen (Houben-Weyl, Bd. 11/1, S. 341ff). Dabei können als Lösungsmittel organische Säuren, wie beispielsweise Essigsäuren, anorganische Säuren, wie beispielsweise HCl, oder organische Lösungsmittel, wie Acetonitril, Alkohole, Ether, Ester, Kohlenwasserstoffe, CS₂, in Abhängigkeit des verwendeten Reduktionsverfahrens, zum Einsatz kommen. Die Reaktionstemperatur liegt zwischen -20 °C und +120 °C, vorzugsweise zwischen +20 °C und +60 °C. Die Zugabe des Reduktionsmittels kann in überstöchiometrischen (> 1 Äquivalent bezogen auf IV), stöchiometrischen (1 Äquivalent bezogen auf IV) und in katalytischen Mengen erfolgen.

Zur katalytischen Reduktion können handelsübliche Katalysatoren eingesetzt werden, wie beispielsweise Pd/C, Rh/Al₂O₃, Pt/C, Raney-Nickel, Raney-Kobalt, Kupferchromit, Platinoxid, Palladiumhydroxyd. Die hydrogenolytische Reduktion kann bei Normaldruck oder Drucken bis zu 50 atm erfolgen.

Bevorzugt erfolgt die Reduktion in Gegenwart von CS₂, Zink oder hydrogenolytisch mit Pd/C, Pt/C oder Ra-Ni. Besonders bevorzugt ist die katalytische Hydrierung von IV mit R³ = OH mit Pd/C, welche ggf. in salzsaurer Lösung bei Normaldruck und Raumtemperatur, eventuell unter Zusatz von Ethanol durchgeführt werden kann.

Die Hydrolyse von Verbindungen der allgemeinen Formel (IV) mit R³ = H zu den L- bzw. D-Aminosäure-Derivaten mit R³ = H kann analog der WO 97/10203 in einem sauren Reaktionsmilieu erfolgen, wie beispielsweise in Gegenwart einer anorganischen Säure, wie HCl, HBr oder H₂SO₄, und/oder einem sauren Kationenaustauscher und/oder einer organischen Säure, wie para-TsOH, Camphersulfonsäure, Bromchamphersulfonsäure oder Essigsäure, und/oder einem organischen Lösungsmittel, wie Toluol oder Methanol. Die Reaktion kann bei Temperaturen zwischen 0 °C und 140 °C erfolgen, bei Normaldruck oder auch im Autoklaven. Die Aufarbeitung und Isolation erfolgt in üblicher Weise und analog D. Seebach, R. Fitzi, Tetrahedron 44 (1988) 5277.

Ganz besonders bevorzugt ist die Variante, bei der man die Hydrolyse in wäßriger Salzsäure durchführt.

Durch geeignete Reaktionsführung in Abhängigkeit von Temperatur und Säurekonzentration kann man entweder optisch aktive α-Aminosäureamide oder die entsprechenden α-Aminosäuren erhalten (vgl. D. Seebach, E. Juaristi, D. Müller, Ch. Schickli und Th. Weber, Helv. Chim. Acta, 70 (1987), 237) .

Die Hydrolyse der Verbindungen der allgemeinen Formel IV mit R³ = OH zu den L- bzw. D-Hydroxylaminosäurederivaten der Formel I mit R³ = OH und X = NH erfolgt analog der WO 97/10203, kann aber sehr viel einfacher in einem Reaktionsschritt mit einer alkoholischen Lösung einer Mineralsäure, besonders bevorzugt mit HCl in Ethanol unter milden Bedingungen, bes. bevorzugt bei Raumtemperatur durchgeführt werden.

Die Verbindungen der allgemeinen Formel I können dabei je nach den gewählten Hydrolysebedingungen auch in Form eines Säureadditionssalzes, wie beispielsweise als HCl-Salz, HBr-Salz, H₂SO₄-Salz, para-TsOH-Salz, Camphersulfonsäure-Salz, Bromcamphersulfonsäure-Salz oder Essigsäure-Salz, anfallen.

Optional lassen sich die Verbindungen der allgemeinen Formel (IV), worin R¹ und R² die oben angegebene Bedeutung annehmen und R³ = OH ist, zu Verbindungen der allgemeinen Formel V 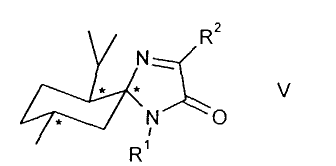 worin R¹ und R² die oben angegebene Bedeutung annehmen, eliminieren, anschließend reduzieren und zu den Verbindungen der allgemeinen Formel (I) mit R³ = H hydrolysieren.

Die Dehydratisierung der Verbindungen IV kann in an sich bekannter Weise (Houben-Weyl "Methoden der Organischen Chemie", Bd 10/1, S. 1247, Bd E16a, S. 211ff) erfolgen. So wird beispielsweise die Hydroxylamin-Verbindung des Typs IV in einem organischen Lösungsmittel, wie Methylenchlorid, Pyridin, Ether, in Gegenwart eines wasserentziehenden Mittels, wie N,N'-Carbonyldiimidazol, DCC oder P₂O₅, unter Luftausschluß (z. B. N₂-Atmosphäre oder Argon-Atmosphäre) bei Temperaturen zwischen 0 °C und 120 °C, vorzugsweise zwischen 20 °C und 30 °C, gerührt und nach Beendigung der Reaktion aufgearbeitet.

Die Eliminierungsprodukte V können dann wie erwähnt unter reduzierenden Bedingungen und Konfigurationsumkehr in die optischen Antipoden des ursprünglichen Typs IV mit R³ = H überführt werden. Die Reduktion kann analog der bereits beschriebenen Reduktion von Verbindungen des Typs IV mit R³ = OH zu IV mit R³ = H durchgeführt werden. Bevorzugt ist die katalytische Hydrierung mit Pd(OH)₂/C in Ethanol bei 25 °C und Normaldruck (vgl. auch B. Trost, I. Fleming, Compr. Org. Syn., Bd. 8, "Reductions", Pergamon Press, Oxford 1991).

Die sich anschließende Hydrolyse von den optischen Antipoden von IV mit R³ = H zu Verbindungen des Typs I mit R³ = H erfolgt analog wie oben beschrieben.

Es ist darüber hinaus möglich, wie in der WO 97/10203 beschrieben, die Verbindungen des Typs IV 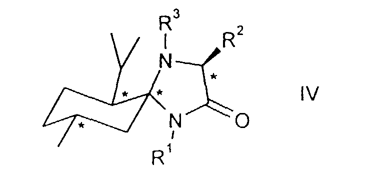 mit R³ = OH wiederum zu Nitronen zu oxidieren und erneut einer weiteren Umsetzung mit Nucleophilen zugänglich zu machen. Man erhält so in einem weiteren Reaktionsschritt optisch aktive α,α-disubstituierte Aminosäurederivate der allgemeinen Formel IV.

Die weiteren Umsetzungen von den so erhältlichen α,α-Dialkylderivaten erfolgt analog der oben erwähnten Maßnahmen.

Die Herstellung der Verbindung der allgemeinen Formel (II) ist in der WO 97/10203 beschrieben, kann aber vorteilhafter unter Umgehung von chlorkohlenwasserstoffhaltigen Lösungsmitteln und halogenhaltigen, aromatischen Persäuren wie 3-Chlorperoxobenzoesäure in Alkoholen unter Verwendung von MMPP (Monoperoxyphthalsäure-Magnesiumsalz) oder mittels Wasserstoffperoxyd in Gegenwart von katalytischen Mengen an Methyltrioxorhenium (MeReO₃) durchgeführt werden.

Das Hydrazin (III) kann nach dem Fachmann bekannten Verfahren hergestellt werden (Houben-Weyl, Band E16a, S. 425ff, Hrsg. D. Klamann und X/2, S. 1ff, Hrsg. E. Müller).

Die Umsetzung von Verbindungen des Strukturtyps II zu Verbindungen der allgemeinen Formel IV erfolgt unter Zusatz von monosubstituiertem Hydrazin III z. B. in Anwesenheit eines Oxidationsmittels. Die diastereomerenreinen Verbindungen IV fallen nach Reinigung durch Kristallisation, Destillation oder Säulenchromatographie in Ausbeuten bis 96 % an.

Dazu werden die Verbindungen des Strukturtyps II in einem organischen Lösungsmittel, beispielsweise Toluol, Essigester, Ether, Alkoholen, etc. gelöst und bevorzugt bei Raumtemperatur mit 3-6 Äquivalenten eines monosubstituierten Hydrazins III und 3-6 Äquivalenten eines Oxidationsmittels versetzt.

Alternativ kann das Hydrazin III aus den Hydrazinsäureverbindungen durch Base, beispielsweise Natriumhydroxid, Kaliumhydroxid oder Triethylamin, in situ freigesetzt werden.

Nach Beendigung der Reaktion, leicht an der sich abschwächenden Stickstoffentwicklung erkennbar, wird je nach verwendeten Oxidationsmitteln einfach der feste Rückstand abfiltriert beziehungsweise wäßrig aufgearbeitet (Abtrennung und Extraktion der wäßrigen Phase mit einem entsprechenden organischen Lösungsmittel und Trocknung der gesammelten organischen Phasen). Die Reinigung der Rohprodukte erfolgt durch Umkristallisation, Destillation oder Säulenchromatographie.

Ein wesentlicher Vorteil des Verfahrens ist, daß die Ausgangsstoffe (+)-Menthol als auch (-)-Menthol kommerziell zu günstigen und ähnlichen Preisen problemlos erhältlich sind.

Das neue Verfahren erlaubt gegenüber den bekannten Verfahren eine neuartige Reaktionsführung, die eine einfachere und schnellere Herstellung der Verbindungen des allgemeinen Typs I ermöglicht. Je nach gewähltem Ausgangsmaterial können so gezielt L-α-Hydroxylaminosäure- oder L-α-Aminosäurederivate oder D-α-Hydroxylaminosäure- oder D-α-Aminosäurederivate erhalten werden.

Es können vorteilhafterweise ausgehend von (-)-Menthol die entsprechenden L-α-Hydroxylaminosäuren/L-α-Aminosäuren bzw. ihre Derivate und ausgehend von dem optischen Antipoden (+)-Menthol die entsprechenden D-α-Hydroxylaminosäuren/D-α-Aminosäuren bzw. ihre Derivate erhalten werden. Die Kennzeichnung der asymmetrischen Kohlenstoffe im Cyclohexanfragment der Verbindungen der allgemeinen Formeln II und IV mit * soll deutlich machen, daß es sich hierbei um ein Stereozentrum der Konfiguration 6S,9R oder 6R,9S, je nach gewähltem Ausgangsmaterial (-)-Menthol oder (+)-Menthol handelt. Die eindeutige und absolute Konfiguration des weiteren Asymmetriezentrums in IV ergibt sich aus der Wahl der Ausgangsstoffe. So wird das Zentrum am α-C-Atom des Aminosäurebausteins in IV eindeutig durch die Konfiguration am Cyclohexanring festgelegt.

(-)-Menthol bzw. (+)-Menthol wird dabei in an sich literaturbekannter Weise (Houben-Weyl "Methoden der Organischen Chemie", Bd. 7/2a, S. 724) durch Oxidation in das entsprechende (-)-Menthon bzw. (+)-Menthon überführt.

Die hier vorgeschlagene radikalische Addition an das Nitron (II) ermöglicht im Vergleich zur bisher bekannten radikalischen Addition mit anschließender Hydrierung eine effizientere Synthese von z. B. optisch aktivem tert.-Leucin. Neben der Verkürzung der Reaktionssequenz um eine Reaktionsstufe, verglichen mit der radikalischen Addition der WO 97/10203, verläuft die Reaktion hier in wesentlich höheren Ausbeuten. Ebenfalls ist keinerlei Kühlung zur Kontrolle der Reaktionstemperatur notwendig, wie bei der Reduktion des substituierten Nitrons (II) mit Lithiumaluminiumhydrid in der WO 97/10203. Durch die weite Variabilität des Oxidationsmittels zur Erzeugung der Radikale kann vorteilhafterweise entgegen der bekannten Variante auf Schwermetalle verzichtet werden.

Im Vergleich zu den beschriebenen ionischen Verfahren ist auf die erhöhten Ausbeuten der radikalischen Addition, besonders bei sterisch anspruchvollen Resten, hinzuweisen. Im Falle des *tert*.-Butylrestes ergibt sich eine Steigerung der Ausbeute von 40 % auf über 96 %, bei ebenfalls gleich hoher Diastereoselektivtät.

Auf komplizierte Reaktionstechniken, wie Wasserausschluß, tiefe Reaktionstemperaturen und schwer handhabbare, metallorganische Reagezien kann vollständig verzichtet werden. Stattdessen sind die Reaktionsbedingungen, das Lösungsmittel sowie das verwendete Oxidationsmittel in weiten Bereichen frei wählbar. Im Falle einer elektrochemischen Oxidation des Hydrazins läßt sich sogar die Salzbildung minimieren. Alle bekannten Nebenprodukte, z.B. Stickstoff, Alkane, Kaliumsulfat etc. sind im industriellen Maßstab unbedenklich zu handhabende Reaktionsprodukte.

Die eingesetzten Edukte sind kommerziell erhältlich oder einfach synthetisierbar

Unter der Bezeichnung "Alkyl" sind sowohl "geradkettige" als auch "verzweigte" Alkylgruppen zu verstehen. Unter der Bezeichnung "geradkettige Alkylgruppe" sind beispielsweise Reste wie Methyl, Ethyl, n-Propyl, n-Butyl, n-Pentyl, n-Hexyl, unter "verzweigter Alkylgruppe" Reste wie beispielsweise Isopropyl, Neopentyl oder tert.-Butyl zu verstehen. Die Bezeichnung Halogen steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy samt deren mögliche Bindungsisomere dar.

Die folgenden Beispiele sollen die Erfindung erläutern:

### A) Oxidation zur Verbindung der allgemeinen Formel II unter Einsatz von Magnesiummonoperoxyphthalsäure, MMPP

(0,66 mol) der Verbindung des Typs II der WO 97/10203 werden in einer Mischung aus 2.50 L EtOH (techn.) und 0.50 L destilliertem H₂O (v/v = 5 : 1) vorgelegt und unter Rühren und kontinuierlicher Zugabe über eine Feststoffschnecke mit 403 g (0.774 mol) Magnesiummonoperoxyphthalsäure Hexahydrat (85 proz.) bei einer Temperatur von 35-45 °C innerhalb 1.5 h langsam versetzt. Es zeigt sich nun durch GC-Analyse eine Transformation zum gewünschten Endprodukt von bereits 70 %. Eine DC-Analyse zeigt das entsprechende Hydroxylamin als Zwischenprodukt und läßt kein Edukt mehr erkennen. Die Suspension rührt bei nicht höher als 45 °C über Nacht. Iod-Stärke-Papier zeigt als Schnelltester die An- oder Abwesenheit von verfügbarem Sauerstoff an. Nach vollständiger Reaktion wird überschüssiges Oxidationsmittel mittels ges. Na₂SO₃-Lösung reduziert und die Reaktionslösung bis zur Trockene im Vakuum eingedampft. Anschließend wird der Rückstand in Diethylether / 5proz. NaOH aufgenommen, die Phasen getrennt und noch zweimal mit Diethylether extrahiert. Die vereinigte organische Phase wird jeweils einmal mit ges. NaHCO₃-Lösung und ges. NaCl-Lösung gewaschen, über MgSO₄ getrocknet und eingedampft.

Das NMR-Rohspektrum zeigt im Falle der Verbindung der allgemeinen Formel II mit R¹ = Me bereits eine hohe Reinheit des Roh-Produktes (GC-%: 98.7), daß zu 146 g (93 %) farblos kristallin isoliert wird. Es kann aus Diethylether umkristallisiert werden. Smp.: 125 °C

### B) Oxidation zur Verbindung der Formel II unter Einsatz von Wasserstoffperoxyd und einer katalytischen Menge an Methyltrioxyrhenium, MTO

(0.90 mmol) der Verbindung des Typs II der WO 97/10203 werden in 20 mL EtOH (techn.) vorgelegt und unter Rühren mit 0.35 g ≡ 0.31 mL 35proz. aq H₂O₂ (3.60 mmol) und 9 mg (0.036 mmol ≡ 4 mol %) Methyltrioxyrhenium bei RT versetzt. Es wird bei RT über Nacht gerührt. Nach vollständiger Reaktion wird ges. Na₂SO₃-Lösung zur Reduktion von überschüssigem Oxidationsmittel (Iod-Stärke-Papier zeigt die Vollständigkeit an) zugesetzt. Durch Eluierung über eine Kieselgel-Flash-Säule wird das MTO absorptiv entfernt. Es wird zweimal mit jeweils 15 mL EtOH nachgewaschen. Das Eluat wird im Vakuum bis zur Trockene eingedampft und in Diethylether / H₂O aufgenommen. Die wäßrige Phase wird noch zweimal mit 15 mL Diethylether extrahiert, die vereinigte organische Phase mit ges. NaCl-Lösung gewaschen, über NaSO₄ getrocknet und im Vakuum bis zur Trockene eingedampft.

Im Falle von II mit R¹ = Me wird ein farbloser kristalliner Feststoff als Rohprodukt in 195 mg (92 %) erhalten.

### C) Umsetzung der Verbindung des Strukturtyps II zu einer Verbindung des Typs IV am Beispiel der Darstellung von (3S,5S,6S,9R)-3-tert-Butyl-4-hydroxy-6-isopropyl-1,9-dimethyl-1,4-diazaspiro[4.5]decan-2-on

Jeweils 100 ml einer Lösung von 50,0 g (0,21 mol) II (R¹ = Me) in 1000 ml Toluol, 340,6 g (1,26 mol) Kaliumperoxodisulfat in 1000 ml Wasser und 157 g (1,26 mol) tert-Butylhydrazinhydrochlorid in 1000 ml 10 % Natronlauge werden in einen 4 l Mehrhalskolben gegeben und stark gerührt. Anschließend werden alle 30 min je weitere 100 ml der drei Lösungen gleichzeitig zugegeben, wobei als Reaktionskontrolle jeweils auf die einsetzende Stickstoffentwicklung geachtet wird. 30 min nach der letzten Zugabe wird die organische Phase abgetrennt und die wäßrige Phase zweimal mit ca. 300 ml Toluol gewaschen. Die vereinigten organischen Phasen werden noch zweimal mit Wasser gewaschen und mit Natriumsulfat getrocknet. Nach Entfernen des Lösungsmittels im Vakuum erhält man 53,4 g leicht gelblich gefärbtes Rohprodukt (Ausbeute = 85,8 %), das laut NMR-Spektren rein vorliegt.

Durch Umkristallisation aus Cyclohexan werden 43,4 g (0,147 mol) farblose Kristalle erhalten (Ausbeute = 69,9 %).

Gesamtausbeute nach Nachkristallisation: 48 g (77,2 %)

| | | |
|---|---|---|
| R² | Ausbeute ( % ) | Smp ( °C ). |
| CH₂CH₃ | 41 (60 Umsatz) | 176,7 °C |
| C(CH₃)₃ | 96 | 171,8 (Zersetzung) |
| C₆H₅ | 71 | * |

| | | |
|---|---|---|
| *¹H-NMR (400.1 MHz, CDCl₃): δ = 7,25-7,50 (m, 5 H, aromat. C-H); 4.84 (s, 1 H, NOH); 4,80 (s, 1 H, CH-C3); 2,85 (s, 3 H, NCH₃); 0,85 - 2.1 (m, 18 H, Menthyl-CHₓ) darunter 1.06 (d, 3 H, CH₃-C11/13/14, ³J = 6.6 Hz); 0.99 (d, 3 H, CH₃-C11/13/14, ³J = 6,6 Hz); 0.97 (d, 3 H, CH₃-C11/13/14, ³J = 6.6 Hz). | | |

### D) Elektrochemische radikalische Alkylierung der Verbindung der allgemeinen Formel II

1,0 g Verbindung des Typs II (R¹ = Me) (4,20 mmol) werden in 50 mL Essigsäureethylester (techn.) vorgelegt und mit 500 mg tert-Butylhydrazinhydrochlorid (4,00 mmol), sowie 500 mg NaOH (12,50 mmol) in 5 mL MeOH versetzt. Die Elektrolyse wird in einer ungeteilten Zelle durchgeführt. Als Elektroden werden eine Kohlestab-Anode (ca. 6 cm²) und eine Kohlestab-Kathode (ca. 6 cm²) verwendet. An der Arbeitselektrode (Anode) wird ein Potential von min. 550 mV bis max. 1000 mV vs. Ag/AgCl/KCl angelegt.
Im Verlauf der Elektrolyse werden drei Mal nach je 24 h jeweils weitere 500 mg tert-Butylhydrazinhydrochlorid sowie 500 mg NaOH in 5 mL MeOH zugegeben. Nach vollständiger Reaktion wird das ausgefallene NaCl abgesaugt. Die organische Phase wird mit H₂O gewaschen und die Lösungsmittel im Vakuum entfernt. Es wird über eine Kieselgel-Säule mit einer 2:1 Mischung von Cyclohexan:Essigsäureethylester eluiert. Nach Eindampfen bis zur Trockene im Vakuum werden 1050 mg (87,5 %) eines farblos kristallinen Feststoffes erhalten.

### E) Darstellung von (2S,5R,6S,9R)-3-tert-Butyl-6-isopropyl-4,9-dimethyl-1,4-diazaspiro[4.5]decan-3-on-hydrochlorid

43,4 g (0,147 mol) der Verbindung der allgemeinen Formel IV (R¹ = Me, R² = tBu, R³ = OH) werden in 200 ml Ethanol gelöst und mit 200 ml 1,5 N HCl-Lösung und 20 g Pd/C-Katalysator versetzt. Man evakuiert kurz den Kolben, begast mit Wasserstoff und rührt die Suspension 3 Tage.
Anschließend wird der Katalysator abfiltriert und mehrmals intensiv mit 1 N HCl und Ethanol gewaschen. Die gesammelten Filtrate werden einrotiert, und man erhält 49,4 g eines farblosen, leicht feuchten Rohprodukts (Ausbeute = 106 %), das laut NMR-Spektren rein vorliegt.

### F) Freisetzung von L-tert-Leucin-methylamid-Hydrochlorid

Die 49,4 g des Produktes aus Versuch E werden in 1200 ml 1 N HCl und 150 ml Eisessig suspendiert und 12 h unter Rückfluß gekocht. Nach Entfernen des Lösungsmittels im Vakuum erhält man 25,6 g (0,142 mol) eines farblosen Feststoffes (Ausbeute = 96,5 %), das laut NMR-Spektren rein vorliegt. Der ee-Wert beträgt 99.5 %.

## Patentansprüche

1. Verfahren zur Herstellung enantiomerenangereicherter Aminosäuren und Aminosäurederivaten der allgemeinen Formel (I) oder Säureadditionssalzen hiervon 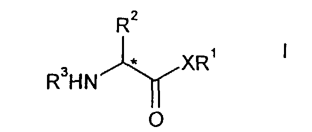 worin
* = Asymmetriezentrum
X = O oder NH
R¹ = H, (C₁-C₆) Alkyl, Benzyl oder
(C₃-C₆) Alkoxycarbonylmethyl und
R² = H, (C₁-C₆) Alkyl, das mit Heteroatomen wie N, P, O, S oder Si unterbrochen oder substituiert sein kann, wobei die Heteroatome selbst ein- oder mehrfach mit (C₁-C₃) Alkyl substituiert sein können, (C₂-C₆) Alkenyl, (C₁-C₆) Haloalkyl, Halogen, Aryl, wie Naphthyl oder Phenyl, welche ein- oder mehrfach mit (C₁-C₃) Alkyl, Hydroxy, Halogen oder (C₁-C₃) Alkoxy substituiert sein können, Aralkyl wie 2-Naphthylmethyl oder Benzyl oder 1,1- oder 1,2-Phenethyl, welche seinerseits ein- oder mehrfach mit (C₁-C₃) Alkyl, Hydroxy, Halogen oder (C₁-C₃) Alkoxy substituiert sein können, Heteroaralkyl wie N-geschütztes 3-Indolylmethyl
R³ = H, OH bedeutet,
aus diastereomerenangereicherten Nitronen der allgemeinen Formel (II) 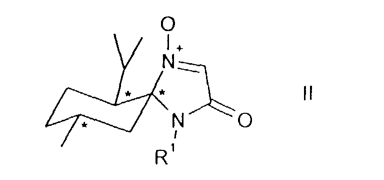 worin *, R¹ die oben angegebene Bedeutung besitzen,
**dadurch gekennzeichnet,**
daß man das Nitron der allgemeinen Formel (II) mit einem Hydrazinderivat der allgemeinen Formel (III) 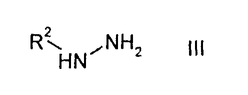 wobei R² die oben angegebene Bedeutung besitzt, in einem Lösungsmittel in Gegenwart eines Radikalstarters oder unter elektrochemischen Bedingungen zu diastereomerenangereicherten Verbindungen der allgemeinen Formel (IV) 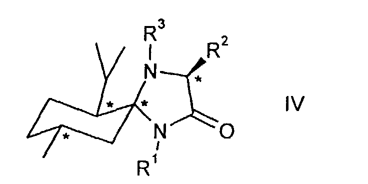 worin R¹ und R² die oben angegebene Bedeutung annehmen und R³ = OH ist, umsetzt, anschließend hydrolysiert oder erst zu Verbindungen der allgemeinen Formel (IV), worin R¹ und R² die oben angegebene Bedeutung annehmen und R³ = H ist, reduziert und dann hydrolysiert.

2. Verfahren Anspruch 1,
**dadurch gekennzeichnet,**
daß man die radikalische Substitution bei Temperaturen zwischen -78 °C und +150 °C durchführt.

3. Verfahren nach einem oder mehreren der vorhergehenden Ansprüche,
**dadurch gekennzeichnet,**
daß man als Lösungsmittel ein Zweiphasensystem aus Toluol/Wasser verwendet.

4. Verfahren nach einem oder mehreren der vorhergehenden Ansprüche,
**dadurch gekennzeichnet,**
daß man als Radikalstarter ein Oxidationsmittel, wie Natrium- oder Kaliumperoxodisulfat, Natriumpercarbonat oder Natriumperborat, verwendet.

5. Verfahren nach einem oder mehreren der vorhergehenden Ansprüche,
**dadurch gekennzeichnet,**
daß man die Reduktion mittels katalytischer Hydrierung mittels Pd/C durchführt.

6. Verfahren nach einem oder mehreren der vorhergehenden Ansprüche,
**dadurch gekennzeichnet,**
daß man die Hydrolyse in einem angesäuerten Lösungsmittel durchführt.

7. Verfahren nach einem oder mehreren der vorhergehenden Ansprüche,
**dadurch gekennzeichnet,**
daß man im Falle von IV mit R³ = H die Hydrolyse in wäßriger Salzsäure durchführt.

8. Verfahren nach einem oder mehreren der vorhergehenden Ansprüche,
**dadurch gekennzeichnet,**
daß man im Falle von IV mit R³ = OH die Hydrolyse mittels Salzsäure in alkoholischer Lösung durchführt.

9. Verfahren nach einem oder mehreren der vorhergehenden Ansprüche,
**dadurch gekennzeichnet,**
daß man das nach der Hydrolyse entstehende Menthon recycliert.

10. Verfahren nach Anspruch 1,
**dadurch gekennzeichnet,**
daß man die Oxidation zu Verbindung der allgemeinen Formel (II) mittels Magnesiummonoperoxyphthalsäure (MMPP) oder unter Verwendung von Methyltrioxorhenium (MeReO₃) in Gegenwart von H₂O₂ durchführt.

11. Verfahren zur Herstellung von enantiomerenangereicherten Aminosäuren oder Säureadditionssalzen der allgemeinen Formel (I) 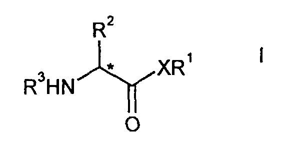 worin
* = Asymmetriezentrum
X = O oder NH
R¹ = H, (C₁-C₆) Alkyl, Benzyl oder
(C₃-C₆) Alkoxycarbonylmethyl und
R² = H, (C₁-C₆) Alkyl, das mit Heteroatomen wie N, P, O, S oder Si unterbrochen oder substituiert sein kann, wobei die Heteroatome selbst ein- oder mehrfach mit linear oder verzweigten (C₁-C₃) Alkyl substituiert sein können, (C₂-C₆) Alkenyl, (C₁-C₆) Haloalkyl, Halogen, Aryl, wie Naphthyl oder Phenyl, welche ein- oder mehrfach mit (C₁-C₃) Alkyl, Hydroxy, Halogen oder (C₁-C₃) Alkoxy substituiert sein können, Aralkyl wie 2-Naphthylmethyl oder Benzyl oder 1,1- oder 1,2-Phenethyl, welche seinerseits ein- oder mehrfach mit (C₁-C₃) Alkyl, Hydroxy, Halogen oder (C₁-C₃) Alkoxy substituiert sein können, Heteroaralkyl wie N-geschütztes 3-Indolylmethyl
R³ = H bedeutet,
**dadurch gekennzeichnet,**
daß man Verbindungen der allgemeinen Formel (IV), welche nach dem Verfahren gemäß Anspruch 1 hergestellt wurden und worin R¹ und R² die oben angegebene Bedeutung annehmen und R³ = OH ist, zu Verbindungen der allgemeinen Formel (V) 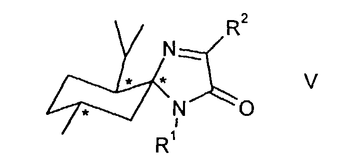 worin R¹ und R² die oben angegebene Bedeutung annehmen, eliminiert, anschließend reduziert und hydrolysiert.
